# EUROPEAN PATENT SPECIFICATION

(11) **EP 2 085 464 B1**
(45) Date of publication and mention of the grant of the patent: **17.11.2010**
(21) Application number: 08001918.5
(22) Date of filing: 01.02.2008
(51) Int. Cl.: C12M 1/24, B01L 3/14, B65D 55/16

(54) **Laboratory vessel with shiftable vessel closure**
Laborgefäß mit verstellbarem Gefäßverschluss
Récipient de laboratoire doté d'une fermeture pouvant être déplacée

(43) Date of publication of application: 05.08.2009
(73) Proprietor: EPPENDORF AG, 22339 Hamburg (DE)
(72) Inventor: Beese, Jochen, 22848 Norderstedt (DE)
(74) Representative: Becker Kurig Straus

(56) References cited:
- EP-A- 0 336 168
- WO-A-97/04073
- WO-A-2007/071287
- US-A- 4 534 483
- US-A- 5 116 758
- US-A1- 2002 130 100

## Description

### Field of the invention

The present invention pertains to vessels with a vessel closure, and in particular to laboratory vessels with a shiftable closure assembly. The laboratory vessel comprises a container comprising an outwardly extending neck with an opening which provides access to the interior of said container, at least one guiding device comprising a guided member and a guiding member, a closure assembly comprising a vessel closure and at least one guided member which is part of said at least one guiding device, which is characterized in that said vessel closure is arranged to be shifted away from said opening to provide access to the interior of the container, wherein said at least one guiding device guides the shifting of the closure assembly and retains it in connection with the container even in the position in which the vessel closure is removed from the opening.

### State of the art

Vessels for a wide range of applications, which are made of various materials and having corresponding closure means have been designed and used. In the laboratory vessels are used for example to culture microorganisms, cell or tissue cultures in a culture medium. Such vessels can have different shapes such as cylindrical bottles, flat round dishes or rectangular flasks.

For example US-P-6,818,438 discloses a tissue culture flask having a generally hexagonal container with a bottom wall from which a ramping extends to one side wall of the flask, from which side wall a substantially tubular neck extends forward therefrom having a opening to provide access into the interior of the container for inserting or removing material, e.g. by using a pipette or scraper.

During the usual laboratory handling of culture vessels it is often required to open and close said vessels, wherein often a large number of flasks have to be handled for example to exchange culture medium, to inoculate cells, or to remove cultured cells etc..

The commonly used vessels often use some form of a threaded vessel closure and a corresponding threaded neck. Opening and closing requires a repeated rotation of the vessel closure with respect to the vessel. Normally this operation is manually done, which requires a certain time and is furthermore, especially when many vessel are handled at a certain time, not the most ergonomic gesture. In addition, for opening and closing these threaded vessel closures often some force must be exerted by the vessel closure on the vessel, so that the vessel closure cannot be opened or closed with a single hand.

In addition to open and close a vessel to provide access into the interior of the container it is also often required to position the vessel closure in a vented position, i.e. the opening of the neck is still covered by the vessel closure, but a gas exchange with the surrounding atmosphere is possible. The change from a closed position into the vented position may for example be achieved by a partial rotation of a threaded vessel closure, so that said vessel closure is still positioned on the neck in the vented position.

As disclosed for example in WO 88/01605 there may be used hydrophobic filter elements with a pore size enabling gas exchange between the interior of the vessel and the surrounding atmosphere but preventing the entrance of contaminating agents in order to prevent a contamination and to maintain the content of the vessel under sterile conditions.

These commonly used threaded vessel closures and threaded necks have several practical problems. Beside the fact that the opening and closing of the vessel requires a repeated rotation of the vessel closure with respect to the vessel during each operation, this operations require the usage of both hands (one for holding the vessel and the other to rotate the vessel closure) as some force has the be exerted by the vessel closure on the vessel.

The usage of both hands is time-consuming, since normally additional instruments for handling e.g. the cultures, such as pipettes or scrapers, are needed so that one hand of the user is occupied by such instruments needed for such operations. In other words, after removing the vessel closure from the neck, said vessel closure has to be stored somewhere else in the meantime so that the user can use this hand e.g. for holding a pipette, and vice versa. Putting away the vessel closure is not advantageous also for sterility reasons. As the handling of cell cultures is often performed under a sterile hood with a vertical laminar flow any object that interrupts the air flow cause a turbulence which may result in the entrance of non sterile surrounding atmosphere. Therefore, having only one arm in the hood or moving the two arms in the hood as less as possible reduces the risk of a contamination. Moving of the arms will be reduced, when one hand does not need to change permanently from rotating a vessel closure to the capture of an additional instrument.

In addition said handling requires a certain training as putting away the vessel closure to store it e.g. on the surface of the hood may result in a contamination of the vessel closure, the user, and/or the hood, if not done carefully. Furthermore, when several vessel are handled, i.e. opened and closed in parallel, a mixing of vessel closure may occur.

US-P-5,398,837 provides another solution for the design of a vessel closure. There is disclosed a cell culture flask having a hinged vessel closure. Said vessel closure can be opened and closed by one hand, wherein the hinged vessel closure is tilted for opening the vessel, i.e. when the vessel closure is positioned to provide access to the interior of the container the vessel closure remains adjacent to the neck at the hinge. This tilted orientation has the disadvantage, that the inner surface of the vessel closure is exposed in the area of the opening of the neck, so that a contact with e.g. a pipette and thus a contamination may occur.

EP 0 336 168 discloses a closure device for a container with an outwardly extending neck. The closure device may be shifted directly in front of the flask's opening guided by a slider. A stopper comprising a spring in an inner cavity and a conical part is urged by the spring to insert itself into the opening of the container, thus sealing the container.

Thus, the problem of the present invention resides in providing a laboratory vessel closable by a vessel closure, wherein the opening and closing of the vessel must be easily possible by using only one hand.

### Summary of the present invention

The above mentioned problems has been solved by a laboratory vessel (10), comprising:
a container comprising an outwardly extending neck (18) with an opening which provides access to the interior of said container;
at least one guiding device (26) comprising of a guided member and a guiding member;
a closure assembly comprising a vessel closure (22) in communication with the guided member,
wherein said vessel closure (22) is arranged to be shifted away from said opening to provide access to the interior of the container, wherein said at least one guiding device (26) guides the shifting of the closure assembly and retains it in connection with the container, characterized in that the vessel closure (22) comprises two lateral segments and the guiding device (26) comprises two further segments (24) as guided member, and the two segments of each pair of segments are arranged opposite to another, wherein the segments are designed such that each of said segments constitutes a quarter of a rim so that in the open position of the vessel closure (22) two quarter sections of the rim are recessed and wherein said segments surround the outer side surface of said neck (18) in a closed position, and in that said neck (18) is flattened in the areas in which said segments of the vessel closure (22) are located in the open position, and wherein said vessel closure (22) is arranged such that it can be rotated to change from a closed to an open position, and vice versa, from which open position said vessel closure (22) can be shifted away from said opening to provide access to the interior of the container, and wherein releasable guiding and locking means between the lateral segments of the vessel closure (22) and the neck (18) are provided, which releasable guiding and locking means comprise a threaded segment at the inner surface of said lateral segments of said vessel closure (22) facing towards the outer side surface of said neck (18), which comprises a corresponding thread or a corresponding at least one threaded neck segment.

### Short description of the drawings

Figure 1 shows a preferred embodiment of a laboratory vessel with the lid orientated for left handers.
Figure 2 shows the preferred embodiment of a laboratory vessel with the lid orientated for right handers.
Figure 3 shows the preferred embodiment of a laboratory vessel with the lid in the closing position.
Figure 4 shows the preferred embodiment of a laboratory vessel with the lid closed, the arrow indicating the closing direction.

### Detailed description of the invention

According to a main embodiment of the present invention a laboratory vessel comprises a container comprising an outwardly extending neck with an opening which provides access to

the interior of said container, at least one guiding device comprising of a guided member and a guiding member, and a closure assembly comprising a vessel closure and at least one guided member which is part of said at least one guiding device, wherein the laboratory vessel is characterized in that said vessel closure is arranged to be shifted away from said opening to provide access to the interior of the container, wherein said at least one guiding device guides the shifting of the closure assembly and retains it in connection with the container even in the position in which the vessel closure is removed from the opening.

The laboratory vessel of the present invention may be used for culture purposes, such as the culture of viruses, microorganisms, fungies, tissue cells and/or tissue cultures or for storage purposes, such as the storage of liquids used in an laboratory. According to the purpose of usage the container and the vessel closure have to be made of suitable materials also in accordance with laboratory and safety guidelines. It may be even more preferred that the laboratory vessel is made of (an) autoclaveable material(s) to provide an sterile environment at least inside the vessel by autoclaving the laboratory vessel under suitable conditions known to the skilled person. It may be furthermore preferred that the laboratory vessel of the present invention can be handled by automatic stations.

According to a preferred embodiment of the present invention the laboratory vessel is a culture vessel.

The container may have different shapes and can for example be formed as a cylindrical bottle, a flat round dish or a polygonale flask. The container may be made of any suitable material known to the skilled person, such as glass or suitable plastics, preferably the container is made of polystyrene.

The vessel closure can also have different shapes and dimensions, provided that the vessel closure is designed to be able to completely cover the opening of the neck when being positioned above the opening. Preferably, the vessel closure has a substantially circular cross section. Alternatively or in addition it may be preferred that the vessel closure has a greater cross sectional dimension as the opening of the neck to prevent a contact of the user's hand with the outer side surface of the neck, when the user touches the vessel closure for opening or closing the vessel. Even more preferred the edge(s) of the vessel closure may project from the closure plane, preferably away from the container, in order to expand to contact surface for the user's hand and to prevent that the user touches for example the neck of the container, which may result for example in a contamination of the content of the container. It may be furthermore preferred that the side surface(s) of the vessel closure is/are not completely smooth to provide greater friction for example with the user's fingers, when she/he wishes to open or close the vessel, so that opening and closing of the vessel by only one hand is further facilitated. To provide enhanced friction the side surface of the vessel closure may for example be provided with grooves extending along the side surface(s).

The vessel closure and the closure assembly can be made of any suitable material known to the skilled person, such as plastics, for example polypropylene or polystyrene.

The vessel closure and/or the guiding device may be preferably designed such, that the vessel closure and/or the guiding device can, in case of need, be replaced by new ones.

The guiding device may be any guiding device known to the skilled person, which enables a shifting of the vessel closure in any direction away from the opening of the neck and back thereto to cover it, if desired. The direction of the shifting can be substantially linear or can follow any curved path, as seen in relation to a direct view on the plane of the opening of the neck. The shifting guided by the guiding device may be for example based on a sliding of the at least one guided member or the guiding device may also employ rollers.

The guiding device and the closure assembly may be arranged such that the vessel closure, when shifted away from the neck of the container, does not touch the outer surface of the container. It may be even more preferred that the guiding device comprises one or more arresting members, so that the vessel closure can be fixed in different positions, e.g. completely removed from the opening of the vessel closure or positioned above said opening.

As used herein the term "shift" and/or "shifting" is intended to mean any movement along the plane of the opening of the neck. The vessel closure may be moved substantially parallel to said plane or may be moved along a curved path according to the alignment of the at least one guiding member or in any combination thereof.

The advantage of the shifting of the vessel closure away from or back to the opening of the neck resides inter alia therein that during the complete shifting the inner surface of the vessel closure is orientated away from the user, so that for example the contamination risk of the inner surfaces of the vessel closure and/or the user's hand is reduced. Furthermore the vessel closure can be completely removed from the opening of the vessel so that, for example contrary to a hinged arrangement of a vessel closure, complete access to the interior of the container is provided. By completely removing the vessel closure from the opening the risk of any contact with the vessel closure, for example a contact with a pipette or a scraper, and such the risk of a contamination is greatly reduced. As the vessel closure retains in connection with the container any mixing of vessel closures, in cases where simultaneously more than one vessel are opened, is prevented. Furthermore any contaminations of the vessel closure, the user, and/or a surface of e.g. a hood are prevented, as the vessel closure is not needed to be put away for example on the surface of a hood.

The at least one guiding member may be an additional member or may be according to a preferred embodiment of the present invention be provided by the container of the vessel. It is even more preferred that the at least one guiding member is integrally formed with the container.

According to one embodiment of the present invention the guiding device is designed to enable a shifting of the closure assembly and thus a shifting of the vessel closure away from or back to the opening of the neck in at least two directions in each case. Preferably the vessel closure may be shiftable in at least three directions, even more preferred in at least four directions and still more preferred in at least five directions in each case. This offers the advantage that the vessel according to the present invention may be used by right-handed as well as by left-handed persons and/or that the vessel can be easily opened or closed in different orientations of the vessel.

According to another embodiment of the present invention the laboratory vessel may comprise two guiding devices which are preferably positioned on opposite sides of the neck.

According to a further embodiment of the present invention the at least one guiding device comprises a slider and a track engaging with each other. The at least one slider acts as a guided member and the at least one track acts as a guiding member. Both members may be formed in any suitable manner provided that they correspond to each other and enable a shifting of the vessel closure away from and back to the opening of the neck. For example, the slider may have a substantially rectangular cross section and the track may be a corresponding rectangular groove such that three sides of the slider are at least partially surrounded by the walls of the groove.

According to another embodiment of the present invention the guiding device comprises two sliders being arranged substantially in the same distance to each other and on opposite sides of the neck in the assembled status and two members connecting said sliders and being arranged on opposite sides of the neck in the assembled status, so that a shiftable frame is formed. Said frame provides a more stabile element. It should be understood that said frame can be provided in a substantially planar or in a curved manner to fit for example to a curved side wall of the container.

As used herein an assembled status is intended to mean an status where the closure assembly is already positioned on the container and the at least one guided member is engaged with the at least one guiding member. The vessel closure may either be located to cover the opening of the neck of the container or may be located shifted away from said opening.

According to another embodiment of the present invention the inner surface of the vessel closure which faces towards the plane of the opening of said neck and/or the opening of said neck may additionally or alternatively comprise sealing means to seal the opening of said vessel when the vessel closure is positioned to close the vessel. Thus, the provided sealing of the opening further prevents for example the entrance of contaminating agents and/or inhibits any gas exchange.

As sealing means preferably a projection or a stopper at the inner surface of the vessel closure may be provided, which projection or stopper is adapted to be inserted in the vessel opening to seal said opening, in addition or alternatively the inner surface of the vessel closure may be provided with a groove adapted to receive the front end of the neck when the vessel is closed by the vessel closure. Alternatively or in addition a sealing ring arranged on the front end of said neck and/or on the inner surface of the vessel closure may be provided.

It should be understood that according to this embodiment of the present invention a shifting of the vessel closure away from and/or back to the opening of the neck would be prevented by said sealing means, so that before shifting the vessel closure has to be moved from the inserted position into a shiftable position, i.e. the vessel closure has the be moved away from the plane of the opening so that the engaging sealing means are released and a shifting along the plane of the opening is possible.

According to a further embodiment of the present invention the vessel closure is arranged such that it can be rotated to change from a closed to an open position, and vice versa, from which open position the vessel closure can be shifted away from said opening to provide access to the interior of the container.

Preferably, changing from the closed position to the open position requires a rotation about an angle of about 90°.

As used herein the closed position is intended to mean a position at which the vessel closure rests on the opening of the neck to close it and preferably to seal it, so that for example an gas exchange with the surrounding atmosphere and/or an contamination of the cultured cells inside the container is prevented. The term open position as used herein is intended to mean a position of the vessel closure enabling a gas exchange of the container with the surrounding environment, so that said open position may also be termed as vented position. In the open position the vessel closure is still located above the opening of the neck, so that a direct access to the interior of the flask is prevented. A shifting of the vessel closure away from the opening of the neck is only possible when the vessel closure is rotated in the open position.

A further advantage of the present invention resides therein that the opening of the container may be covered in either a vented or an air-tight position, so that the laboratory vessel according to the present invention may be used for example for a cultivation either under anaerobic conditions or aerobic conditions.

Such a rotatable vessel closure may according to one preferred embodiment comprise a top, which is adapted to cover the opening of the neck, and at least one lateral segment extending from the top outside the neck opening, and wherein said guiding device, preferably the guided member, comprises at least one further segment adapted to provide together with the at least one lateral segment of the vessel closure a complete rim surrounding the outer side surface of said neck in the closed position, while in the open position the at least one segment extending from the top outside the neck opening and the at least one segment of the guiding device are arranged so that the at least one lateral segment is covered by the at least one further segment so that at least one area of said rim is cleared/recessed and shifting of said closure towards this area is enabled. The segments providing a complete rim in the closed position have such an dimension that they cover in the closed position the outer side surface of the neck at least partially so that a direct contact with said surface is prevented so that for example risks of a contamination of the content of the container is further reduced. If desired, said segments may be made of a material enabling to a certain degree an elastic deformation of the segments during the shifting of the vessel closure away from or back to the neck of the container.

Preferably, means for preventing a contact with the lateral segments during rotation of the vessel closure are provided. For example said vessel closure may be designed such that the cross section dimension of the top is greater than the diameter of the rim. Even more preferred the edge(s) of the top of vessel closure may project from the closure plane, preferably away from the container, in order to expand to contact surface for the user's hand and to prevent that the user touches for example the neck of the container, which may result for example in a contamination of the content of the container. It may be furthermore preferred that the side surface(s) of the top of vessel closure is/are not completely smooth to provide greater friction for example with the user's fingers, when she/he wishes to open or close the vessel, so that opening and closing of the vessel by only one hand is further facilitated. To provide enhanced friction the side surface of the vessel closure may for example be provided with grooves extending along the side surface(s).

According to one embodiment of the present invention releasable guiding and locking means between the at least one lateral segment of the vessel closure and the neck are provided. Said releasable guiding and locking means have beneficial effects as they enhance the fixation of the vessel closure in the closed position and they further guide the rotation of the vessel closure and thus the movement of the closure top away or towards the plane of the opening when changing from the open to the closed position and vice versa. In addition, said guiding and locking means prevent an erroneously opening of the vessel.

According to a preferred embodiment said releasable guiding and locking means comprise a threaded segment at the inner surface of said at least one lateral segment of said vessel closure facing towards the outer side surface of said neck, which outer side surface comprises an corresponding thread or a corresponding at least one threaded neck segment for engagement during rotation of the vessel closure and in the closed position of the vessel closure. The thread guides the rotational movement and further ensures that the at least one lateral segment of the vessel closure is correctly spaced from the outer side surface of the neck so that in the closed position of the vessel closure a complete, closed rim is provided. Preferably, sealing means may be provided on the lateral surfaces of the segments which are arranged adjacent in the closed position in order to improve the tightness of the rim surface.

According to another embodiment of the present invention the at least one lateral segment of the vessel closure and the at least one further segment provided by the guiding device comprise guiding means for guiding the shifting of said segments when changing from the closed to the open position, and vice versa.

It is even more preferred that the at least one lateral segment of the vessel closure comprises a slot on the surface facing towards the neck, and that the at least one further segment provided by the guiding device comprises a corresponding projection adapted to engage with said slot.

According to another embodiment of the present invention the vessel closure and the guiding device each comprise two segments and the two segments of each pair of segments are arranged opposite to another. Said segments may be designed such that each of said segments constitutes a quarter of the rim, so that in the open position of the vessel closure two quarter sections of the rim are recessed, wherein the closure assembly may be shifted towards either of said recesses to provide access to the interior of the container.

According to a further embodiment of the present invention the neck may be additionally or alternatively flattened in the at least one area in which said at least one lateral segment extending from the top is located in the open position. The at least one flattened area enhances the gas exchange between the interior of the container and the surrounding atmosphere in the open position of the vessel closure. Preferably, the flattening has been carried out parallel to the direction of the shifting in order to facilitate the movement of the vessel closure above or away from the opening of the neck.

According to another embodiment of the present invention the vessel closure may further comprise a filter located at the inner side of the top of the vessel closure. Said filter may be any filter known to the skilled person suitable to prevent for example the entrance of any contaminating agents, such as viruses, microorganisms, fungies, into the interior of the container. Preferably said filter may be a hydrophobic filter. The pore size of said filter may be chosen to enable a gas exchange between the interior of the container and the surrounding atmosphere but preventing the entrance of contaminating agents. Preferably, the pore size is in the range of 0,1 to 1 µm, more preferred in the range of 0,2 to 0,5 µm and still more preferred in the range of 0,23 to 0,25 µm. Alternatively the top of the vessel closure may be made of a filter material with an appropriate pore size as mentioned above.

According to another embodiment of the present invention the laboratory vessel may comprise a ventilation unit provided in the top of the vessel closure, so that an gas exchange is also possible in the closed position of the vessel closure.

According to a further embodiment of the present invention the container is a substantially hexagonal container having a bottom side, a top side and side walls, wherein the neck extends from a side wall and wherein said closure assembly is adapted to be shifted in two directions away the opening of the neck, wherein the shift direction is substantially parallel to the bottom and/or top side of the container. Such vessels can be positioned and stored in a stacked alignment, wherein the vessel closure can be easily removed and/or replaced above the opening of the neck in the stacked alignment of the vessels.

The vessel according to the present invention may be provided sterile, free of DNase, RNase, or pyrogene or in any combination thereof. Additionally or alternatively the container may be provided with a grading and/or a labelling area.

According to another aspect the present invention pertains to the use of a laboratory vessel as set forth above for the cultivation of viruses, microorganisms, fungies, tissue cells and/or tissue cultures.

The present invention has been mainly discussed based on the use of the vessel according to the present invention as culture vessel. It should be understood that the vessel according to the present invention may also be used for other purposes where an easy opening and closing of the vessel possible by using only one hand is desired and/or wherein a contamination of the inner surfaces of the vessel closure should be prevented and/or where it is desired that the vessel closure retains in connection with the vessel.

In the following and with reference to the accompanied drawings a preferred embodiment of the present invention will be explained by way of example only without limiting the present invention thereto.

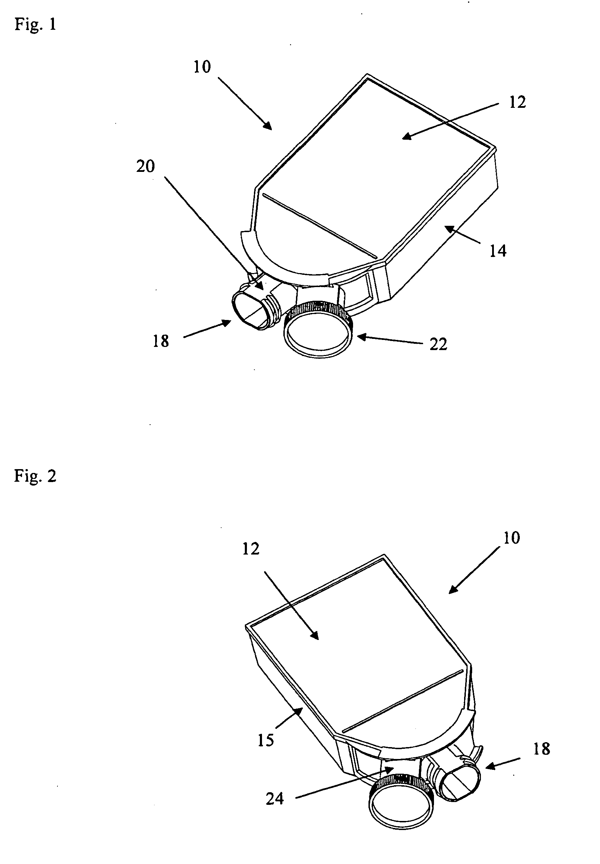

As shown in Figs. 1 and 2, a laboratory vessel (10) according to a preferred embodiment of the present invention comprises a substantially hexagonal container having a bottom side, a top side (12) and side walls. From the two lateral side walls (14, 15) a curved ramp (16) extends to the neck (18) of the vessel. The neck has a substantially circular cross section which is flattened on two sides substantially parallel to the bottom and top side of the container. Said flattenings (20) facilitate the shifting of the vessel closure (22) and also enhance gas exchange in an open position of the vessel closure. The non flattened areas of the neck are each provided with a thread segment on the outer surfaces, which guides for example a movement of the closure vessel away and towards the plane of the opening of the neck.

The closure assembly comprises a vessel closure (22) with a circular top and two lateral segments (24) extending from the inner surface of the top and a guided device (26) comprising two sliders being arranged substantially in the same distance to each other and two members connecting said sliders, so that a shiftable frame is formed which frame further comprises two segments providing parts of the rim of the vessel closure.

The tracks for guiding the shiftable frame are provided by the container, wherein the upper track is integrally formed with the top side of the container and the lower track comprises two fragments integrally formed with the bottom side of the container at opposite sides of the neck. The shiftable frame is curved with the both members connecting the sliders being arranged furthest from the plane of the lateral side walls of the vessel closure. The rim of the vessel closure comprises four segments two extending from the top of the vessel closure and two provided by the guiding device, wherein the two lateral segments extending from the top of the vessel closure are provided with thread segments at the inner surface facing towards the outer surface of the neck.

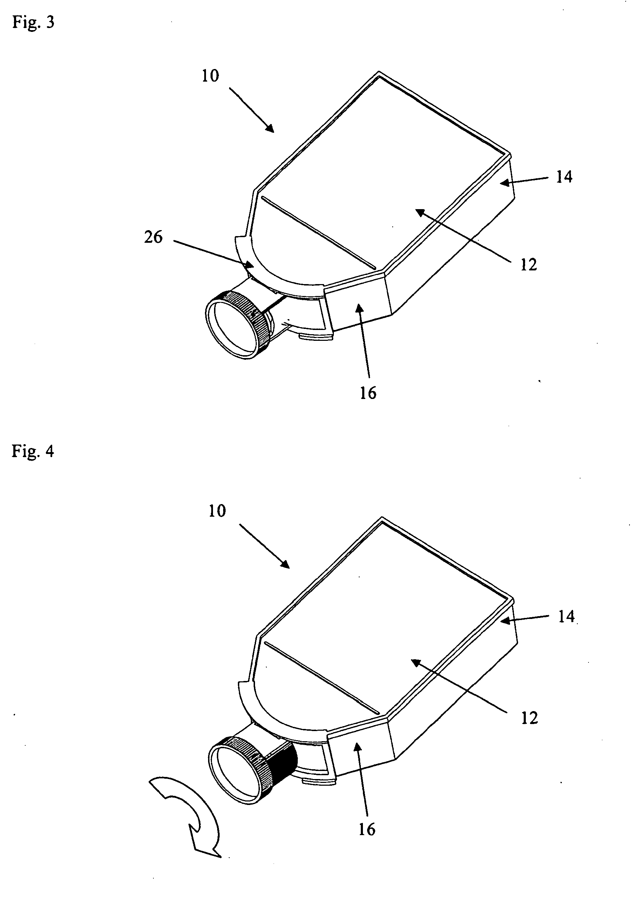

Fig. 3 shows the closing position of the closure assembly, whereas Fig. 4 shows the closure assembly in the closed position with the closing direction of the lid indicated by an arrow. The vessel closure has been rotated by an angle of 90°. Due to the rotation, guided by the thread segments, the top of the vessel closure has been moved on the plane of the opening of the neck, sealingly closing it. As the vessel closure has to be rotated only by an angle of 90° for opening or closing, and not several times as necessary in commonly used threaded necks and lids, the opening and closing of the vessel according to the present invention is less time-consuming.

## Claims

1. Laboratory vessel (10), comprising:
a container comprising an outwardly extending neck (18) with an opening which provides access to the interior of said container;
at least one guiding device (26) comprising of a guided member and a guiding member;
a closure assembly comprising a vessel closure (22) in communication with the guided member,
wherein said vessel closure (22) is arranged to be shifted away from said opening to provide access to the interior of the container, wherein said at least one guiding device (26) guides the shifting of the closure assembly and retains it in connection with the container, **characterized in that** the vessel closure (22) comprises two lateral segments and the guiding device (26) comprises two further segments (24) as guided member, and the two segments of each pair of segments are arranged opposite to another, wherein the segments are designed such that each of said segments constitutes a quarter of a rim so that in the open position of the vessel closure (22) two quarter sections of the rim are recessed and wherein said segments surround the outer side surface of said neck (18) in a closed position, and **in that** said neck (18) is flattened in the areas in which said segments of the vessel closure (22) are located in the open position, and wherein said vessel closure (22) is arranged such that it can be rotated to change from a closed to an open position, and vice versa, from which open position said vessel closure (22) can be shifted away from said opening to provide access to the interior of the container, and wherein releasable guiding and locking means between the lateral segments of the vessel closure (22) and the neck (18) are provided, which releasable guiding and locking means comprise a threaded segment at the inner surface of said lateral segments of said vessel closure (22) facing towards the outer side surface of said neck (18), which comprises a corresponding thread or a corresponding at least one threaded neck segment.

2. Laboratory vessel (10) according to claim 1, wherein the guiding device (26) is designed to enable a shifting of the closure assembly away from or back to the opening of the neck (18) in at least two directions in each case.

3. Laboratory vessel (10) according to claims 1 or 2, wherein the laboratory vessel (10) comprises two guiding devices (26).

4. Laboratory vessel (10) according to any of the proceeding claims, wherein each guiding device (26) comprises a slider and a track engaging with each other.

5. Laboratory vessel (10) according to claims 3 or 4, wherein said guiding device (26) comprises two sliders being arranged substantially in the same distance to each other and on opposite sides of said neck (18) in the assembled status and two members connecting said sliders and being arranged on opposite sides of said neck (18) in the assembled status.

6. Laboratory vessel (10) according to any of the proceeding claims, wherein the inner surface of said vessel closure (22) which faces towards the opening of said neck (18) and/or the opening of said neck (18) comprises sealing means to seal the opening of said vessel (10) when the vessel closure (22) is arranged on the opening of the neck (18).

7. Laboratory vessel (10) according to any of the preceding claims, wherein the lateral segments of the vessel closure (22) and the further segments (24) provided by the guiding device (26) comprise guiding means for guiding the shifting of said segments.

8. Laboratory vessel (10) according to claim 7, wherein the lateral segments of the vessel closure (22) comprise a slot on the surface facing towards the neck (18), and wherein the further segments (24) provided by the guiding device (26) comprise a corresponding projection adapted to engage with said slot.

9. Laboratory vessel (10) according to any of the preceding claims, wherein the vessel closure (22) further comprises a filter located at the inner side of the top of the vessel closure (22).

10. Laboratory vessel (10) according to any of the preceding claims, wherein a ventilation unit is provided in the top of the vessel closure (22).

11. Laboratory vessel (10) according to any of the preceding claims, wherein said container is a substantially hexagonal container having a bottom side, a top side (12) and side walls (14, 15), wherein the neck (18) extends from a side wall (14, 15) and wherein said closure assembly is adapted to be shifted in two directions away the opening of the neck (18), wherein the shift direction is substantially parallel to the bottom and/or top side (12) of the container.

12. Use of a laboratory vessel (10) according to any of the preceding claims for the cultivation of viruses, microorganisms, fungies, tissue cells and/or tissue cultures.

## Patentansprüche

1. Laborgefäß (10), umfassend:
einen Behälter, umfassend einen sich nach außen erstreckenden Hals (18) mit einer Öffnung, die einen Zugang zum Inneren des Behälters bereitstellt;
mindestens eine Führungseinrichtung (26), die ein geführtes Element und ein Führungselement umfasst;
eine Verschlussanordnung, die einen Gefäßverschluss (22) in Verbindung mit dem geführten Element umfasst,
worin der Gefäßverschluss (22) angeordnet ist, um von der Öffnung weggeschoben zu werden, um Zugang zum Inneren des Behälters bereitzustellen, worin die mindestens eine Führungseinrichtung (26) das Wegschieben der Verschlussanordnung führt und sie in Verbindung mit dem Behälter hält,
**dadurch gekennzeichnet, dass** der Gefäßverschluss (22) zwei seitliche Segmente und die Führungseinrichtung (26) zwei weitere Segmente (24) als geführtes Element umfasst, worin die zwei Segmente von jedem Segmentpaar einander gegenüberliegend angeordnet sind, worin die Segmente gestaltet sind, dass jedes der Segmente ein Viertel eines Randes bildet, so dass in der offenen Position des Gefäßverschlusses (22) zwei Viertel-Abschnitte des Randes ausgespart sind, und worin die Segmente die Außenseitenoberfläche des Halses (18) in einer geschlossenen Position umgeben, und **dadurch**, dass der Hals (18) in den Bereichen abgeflacht ist, in denen die Segmente des Gefäßverschlusses (22) in der offenen Position angeordnet sind, und worin der Gefäßverschluss (22) in derartiger Weise angeordnet ist, dass er durch Drehen von einer geschlossenen zu einer geöffneten Position verändert werden kann, und umgekehrt, worin in der offenen Position der Gefäßverschluss (22) von der Öffnung weggeschoben werden kann, um einen Zugang zu dem Inneren des Behälters bereitzustellen, und worin lösbare Führungs- und Verschlussmittel zwischen den seitlichen Segmenten des Gefäßverschlusses (22) und dem Hals (18) bereitgestellt werden, worin die lösbaren Führungs- und Verschlussmittel ein mit einem Gewinde versehenes Segment an der Innenoberfläche der seitlichen Segmente des Gefäßverschlusses (22) umfassen, das in Richtung der äußeren Seitenoberfläche des Halses (18) gerichtet ist, der ein entsprechendes Gewinde oder entsprechend mindestens ein mit einem Gewinde versehenes Halssegment umfasst.

2. Laborgefäß (10) nach Anspruch 1, worin die Führungseinrichtung (26) so gestaltet ist, dass ein Schieben der Verschlussanordnung weg von oder zurück zu der Öffnung des Halses (18) in jedem Fall in mindestens zwei Richtungen ermöglicht wird.

3. Laborgefäß (10) nach Ansprüchen 1 oder 2, worin das Laborgefäß (10) zwei Führungseinrichtungen (26) umfasst.

4. Laborgefäß (10) nach einem der vorstehenden Ansprüche, worin jede Führungseinrichtung (26) einen Schieber und eine Führung umfassen, die miteinander in Eingriff stehen.

5. Laborgefäß (10) nach Ansprüchen 3 oder 4, worin die Führungseinrichtung (26) zwei Schieber umfasst, die im Wesentlichen im gleichen Abstand zueinander und auf gegenüberliegenden Seiten des Halses (18) im zusammengesetzten Zustand angeordnet sind und worin zwei die Schieber verbindende Elemente in dem zusammengesetzten Zustand auf gegenüberliegenden Seiten des Halses (18) angeordnet sind.

6. Laborgefäß (10) nach einem der vorstehenden Ansprüche, worin die innere Oberfläche des Gefäßverschlusses (22), die der Öffnung des Halses (18) zugewandt ist und/oder die Öffnung des Halses (18) Dichtmittel umfasst, um die Öffnung des Gefäßes (10) abzudichten, wenn der Gefäßverschluss (22) auf der Öffnung des Halses (18) angeordnet ist.

7. Laborgefäß (10) nach einem der vorstehenden Ansprüche, worin die seitlichen Segmente des Gefäßverschlusses (22) und die weiteren Segmente (24), die durch die Führungseinrichtung (26) bereitgestellt sind, Führungsmittel zum Führen der Verschiebung der Segmente umfassen.

8. Laborgefäß (10) nach Anspruch 7, worin die seitlichen Segmente des Gefäßverschlusses (22) einen Schlitz auf der Oberfläche umfassen, die in Richtung Hals (18) zugewandt ist, und worin die weiteren Segmente (24), die durch die Führungseinrichtung (26) bereitgestellt werden, einen entsprechenden Vorsprung umfassen, der angepasst ist, mit dem Schlitz in Eingriff zu stehen.

9. Laborgefäß (10) nach einem der vorstehenden Ansprüche, worin der Gefäßverschluss (22) weiterhin einen Filter umfasst, der an dem oberen Ende an der Innenseite des Gefäßverschlusses (22) angeordnet ist.

10. Laborgefäß (10) nach einem der vorstehenden Ansprüche, worin eine Belüftungseinheit in dem oberen Ende des Gefäßverschlusses (22) bereitgestellt ist.

11. Laborgefäß (10) nach einem der vorstehenden Ansprüche, worin der Behälter ein im Wesentlichen hexagonaler Behälter ist, der eine untere Seite, eine obere Seite (12) und Seitenwände (14, 15) aufweist, worin sich der Hals (18) von einer Seitenwand (14, 15) erstreckt und worin die Verschlussanordnung angepasst ist, in zwei Richtungen weg von der Öffnung des Halses (18) geschoben zu werden, und worin die Verschiebungsrichtung im Wesentlichen parallel zu der unteren und/oder oberen Seite (12) des Behälters verläuft.

12. Verwendung eines Laborgefäßes (10) nach einem der vorstehenden Ansprüche für das Kultivieren von Viren, Mikroorganismen, Pilzen, Gewebezellen und/oder Gewebekulturen.

## Revendications

1. Récipient de laboratoire (10) comprenant :
un réservoir comprenant un col (18) s'étendant vers l'extérieur avec une ouverture procurant l'accès à l'intérieur dudit réservoir,
au moins un dispositif de guidage (26) se composant d'un membre guidé et d'un membre guidant,
un assemblage de fermeture comprenant une fermeture de récipient (22) en communication avec le membre guidé,
dans lequel ladite fermeture de récipient (22) est configurée pour être éloignée de ladite ouverture pour procurer l'accès à l'intérieur du réservoir, dans lequel ledit au moins un dispositif de guidage (26) guide le déplacement de l'assemblage de fermeture et le maintient en connexion avec le réservoir,
**caractérisé en ce que** la fermeture de récipient (22) comprend deux segments latéraux et
le dispositif de guidage (26) comprend deux autres segments (24) en tant que membres guidés et les deux segments de chaque paire de segments sont disposés en opposition l'un par rapport à l'autre, dans lequel les segments sont conformés tels que chacun desdits segments constitue un quartier d'une jante de telle sorte qu'en position ouverte de la fermeture de récipient (22) deux sections de quartier de jante sont encastrées et dans lequel lesdits segments entourent la surface extérieure dudit col (18) en position fermée,
et **en ce que** ledit col (18) est applati dans les zones dans lesquelles sont localisés lesdits segments de fermeture de récipient (22) en position ouverte, dans lequel ladite fermeture de récipient (22) est configurée de façon telle qu'elle peut être tournée pour changer d'une position fermée à une position ouverte, et vice versa, position ouverte à partir de laquelle ladite fermeture de récipient (22) peut être éloignée de ladite ouverture pour procurer l'accès à l'intérieur du réservoir, dans lequel des moyens de guidage et de verrouillage libérables sont disposés entre les segments latéraux de la fermeture de récipient (22) et le col (18), moyens de guidage et de verrouillage libérables qui comprennent un segment fileté à la surface interne desdits segments latéraux de ladite fermeture de récipient (22) faisant face à la face externe de la paroi dudit col (18) qui comprend un filetage correspondant ou au moins un segment de col fileté correspondant.

2. Récipient de laboratoire (10) selon la revendication 1 dans lequel le dispositif de guidage (26) conformé pour permettre un déplacement de l'assemblage de fermeture loin de ou vers l'ouverture du col (18) selon au moins deux directions dans chaque cas.

3. Récipient de laboratoire (10) selon les revendications 1 ou 2 dans lequel le récipient de laboratoire (10) comprend deux dispositifs de guidage (26).

4. Récipient de laboratoire (10) selon l'une des revendications précédentes dans lequel chaque dispositif de guidage comprend un curseur et un rail s'engageant l'un dans l'autre.

5. Récipient de laboratoire (10) selon les revendications 3 ou 4 dans lequel ledit dispositif de guidage (26) comprend deux curseurs disposés sensiblement à la même distance l'un de l'autre et sur des côtés opposés dudit col (18) dans l'état assemblé et deux membres connectant lesdits curseurs arrangés sur des côtés opposés dudit col (18) en l'état assemblé.

6. Récipient de laboratoire (10) selon l'une des revendications précédentes dans lequel la surface interne de ladite fermeture de récipient (22) qui fait face à l'ouverture dudit col (18) et/ou l'ouverture dudit col (18) comprend des moyens de scellement pour sceller l'ouverture dudit récipient (10) lorsque la fermeture de récipient (22) est dsiposée sur l'ouverture du col (18).

7. Récipient de laboratoire (10) selon l'une des revendications précédentes dans lequel les segments latéraux de la fermeture de récipient (22) et les segments additionnels (24) procurés par de dispositif de guidage (26) comprennent des moyens guider le déplacement desdits segments.

8. Récipient de laboratoire (10) selon la revendication 7 dans lequel les segments latéraux de la fermeture de récipient (22) comprennent une rainure sur la surface faisant face au col (18) et dans lequel les segments additionnels (24) procurés par le dispositif de guidage (26) comprennent une projection correspondante adaptée pour s'engager dans ladite rainure.

9. Récipient de laboratoire (10) selon l'une des revendications précédentes dans lequel la fermeture de récipient (22) comprend en outre un filtre logé à la surface interne du sommet de la fermeture de récipient (22).

10. Récipient de laboratoire (10) selon l'une des revendications précédentes dans lequel une unité de ventilation est aménagée dans le sommet de la fermeture de récipient (22).

11. Récipient de laboratoire (10) selon l'une des revendications précédentes dans lequel ledit réservoir est un réservoir sensiblement hexagonal comportant une paroi de fond, une paroi supérieure (12) et des parois latérales (14, 15) dans lequel le col (18) s'étend à partir d'une paroi latérale (14, 15) et dans lequel ledit assemblage de fermeture est adapté pour être éloigné de l'ouverture du col (18) selon deux directions, dans lequel la direction d'éloignement est sensiblement parallèle à la paroi de fond et/ou supérieure (12) du réservoir.

12. Utilisation d'un récipient de laboratoire (10) selon l'une des revendications précédentes pour la culture de virus, de microorganismes, de champignons, de cellules de tissus et/ou de cultures de tissus.
